(19) Europäisches Patentamt
European Patent Office
Office européen des brevets

(11) Veröffentlichungsnummer: **0 021 058**
**A2**

(12) # EUROPÄISCHE PATENTANMELDUNG

(21) Anmeldenummer: **80102886.1**

(22) Anmeldetag: **23.05.80**

(51) Int. Cl.³: **C 07 D 513/04,** C 07 D 333/70, A 61 K 31/54 // (C07D513/04, 333/00, 279/00)

(30) Priorität: **13.06.79 CH 5528/79**
**25.03.80 CH 2325/80**

(43) Veröffentlichungstag der Anmeldung: **07.01.81 Patentblatt 81/1**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Pfister, Rudolf, Dr., Neubadstrasse 128, CH-4054 Basel (CH)**

(74) Vertreter: **Lederer, Franz, Dr. et al, Patentanwälte Lederer, Meyer Lucile-Grahn-Strasse 22, D-8000 München 80 (DE)**

(54) **Benzothienothiazinderivate, deren Verwendung, Verfahren und Zwischenprodukte zu deren Herstellung und diese enthaltende Arzneimittel.**

(57) Benzothienothiazinderivate der allgemeinen Formel

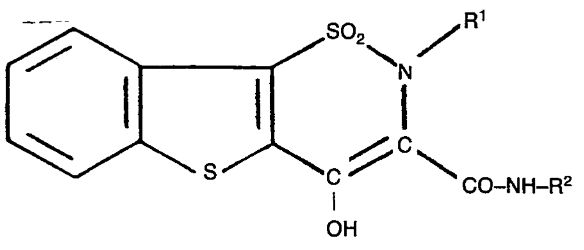

worin $R^1$ niederes Alkyl und $R^2$ den Rest eines gegebenenfalls durch eine oder zwei niedere Alkylgruppen substituierten aromatischen Heterocyclus mit 1 bis 4 Heteroatomen oder einen gegebenenfalls durch Halogen, Hydroxy, niederes Alkyl, Trifluormethyl, Nitro oder niederes Alkoxy substituierten Phenyl- oder Benzylrest bedeuten, sowie pharmazeutisch anwendbare Salze davon sind neu und eignen sich zur Bekämpfung bzw. Verhütung von Entzündungen, Schmerzen, Rheuma und Thrombosen.

Sie können nach verschiedenen Methoden ausgehend von teilweise neuen Ausgangsprodukten hergestellt und in galenische Darreichungsformen gebracht werden.

EP 0 021 058 A2

F.Hoffmann-La Roche & Co.Aktiengesellschaft, Basel/Schweiz

0021058

RAN 4070/55

# Benzothienothiazinderivate, deren Verwendung, Verfahren und Zwischenprodukte zu deren Herstellung und diese enthaltende Arzneimittel.

Die vorliegende Erfindung betrifft Thiazinderivate. Im speziellen betrifft sie Benzothienothiazinderivate der allgemeinen Formel

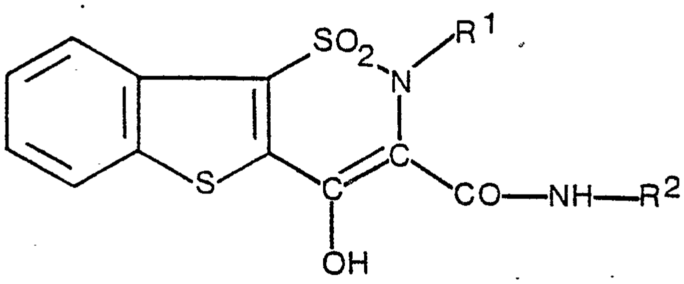

I

worin $R^1$ niederes Alkyl und $R^2$ den Rest eines gegebenenfalls durch eine oder zwei niedere Alkylgruppen substituierten aromatischen Heterocyclus mit 1 bis 4 Heteroatomen oder einen gegebenenfalls durch Halogen, Hydroxy, niederes Alkyl, Trifluormethyl, Nitro oder niederes Alkoxy substituierten Phenyl- oder Benzylrest bedeuten.

Diese Verbindungen sind neu und zeichnen sich durch wertvolle pharmakodynamische Eigenschaften aus.

Gegenstand der vorliegenden Erfindung sind Verbindungen der allgemeinen Formel I und pharmazeutisch anwendbare Salze davon als solche und als pharmazeutische Wirkstoffe, die Herstellung dieser Verbindungen und Zwischenprodukte für die Herstellung dieser Verbindungen, ferner Arznei-

Bt/18.3.80

mittel, enthaltend eine Verbindung der allgemeinen Formel I oder ein pharmazeutisch anwendbares Salz davon, und die Herstellung solcher Arzneimittel, sowie die Verwendung von Verbindungen der allgemeinen Formel I oder von pharmazeutisch anwendbaren Salzen davon bei der Bekämpfung bzw. Verhütung von Krankheiten.

Der in dieser Beschreibung verwendete Ausdruck "niederes Alkyl" bezeichnet geradkettige oder verzweigte gesättigte Kohlenwasserstoffgruppen mit 1-4 Kohlenstoffatomen, wie z.B. Methyl, Aethyl, Propyl, Isopropyl, t-Butyl und dgl. Der Ausdruck "niederes Alkoxy" bezieht sich auf Alkyloxygruppen mit bis zu 4 C-Atomen im Sinne der vorstehenden Definition des Ausdrucks "niederes Alkyl". Die Bezeichnung "Halogen" bezieht sich auf die 4 Halogene Chlor, Brom, Fluor und Jod. Der Begriff "Rest eines gegebenenfalls durch eine oder zwei niedere Alkylgruppen substituierten aromatischen Heterocyclus mit 1-4 Heteroatomen" umfasst gegebenenfalls durch eine oder zwei niedere Alkylgruppen substituierte Reste von 5- oder 6-gliedrigen aromatischen Heterocyclen mit 1-4 Stickstoff- und/oder Sauerstoff- und/oder Schwefelatomen, wie 2-Thiazolyl, 4-Methyl-2-thiazolyl, 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl und dgl..

Der Begriff "gegebenenfalls durch Halogen, Hydroxy, niederes Alkyl, Trifluormethyl, Nitro oder niederes Alkoxy substituierter Phenyl- oder Benzylrest" umfasst Reste wie Phenyl, 4-Hydroxyphenyl, 3-Tolyl, 3-Chlorphenyl, 4-Fluorphenyl, 3-Trifluormethylphenyl, 2,4-Dichlorphenyl, 4-Bromphenyl, 4-Chlorphenyl, 4-Nitrophenyl, 2-Tolyl, 2,5-Dichlorphenyl, 4-Nitro-2-tolyl, 4-Jodphenyl, 4-n-Butylphenyl,

Benzyl, 2-Chlorphenyl und dgl..

In einer bevorzugten Klasse von Verbindungen der Formel I ist $R^1$ eine Methylgruppe. $R^2$ stellt vorzugsweise einen gegebenenfalls durch niederes Alkyl monosubstituierten Pyridylrest dar, wobei der 2-Pyridylrest und der 6-Methyl-2-pyridylrest besonders bevorzugt sind; eine weitere repräsentative Bedeutungsmöglichkeit für das Symbol $R^2$ ist der Phenylrest.

Eine ganz besonders bevorzugte Verbindung der Formel I ist das 4-Hydroxy-2-methyl-N-(2-pyridyl)-2H-[1]benzothieno[2,3-e]-1,2-thiazin-3-carboxamid-1,1-dioxid.

Weitere repräsentative Verbindungen der Formel I sind das 4-Hydroxy-2-methyl-N-(6-methyl-pyridyl-2)-2H[1]benzothieno[2,3-e]-1,2-thiazin-3-carboxamid-1,1-dioxid und das 4-Hydroxy-2-methyl-N-phenyl-2H[1]benzothieno[2,3-e]-1,2-thiazin-3-carboxamid-1,1-dioxid.

Die Benzothienothiazinderivate der Formel I und ihre pharmazeutisch anwendbaren Salze können erfindungsgemäss dadurch hergestellt werden, dass man

a) eine Verbindung der allgemeinen Formel

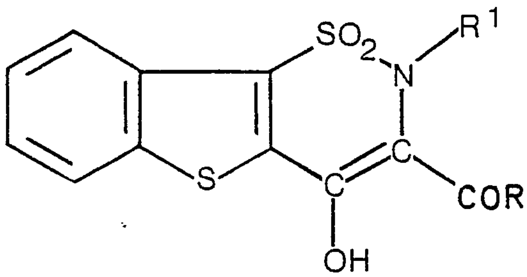

II

worin $R^1$ obige Bedeutung besitzt und R niederes Alkoxy oder einen Rest der Formel $-NR^3R^4$ bedeuten, wobei $R^3$ und $R^4$ je Wasserstoff oder niederes Alkyl oder zusammen mit dem Stickstoffatom einen Heterocyclus bedeuten,

mit einem Amin der allgemeinen Formel

$$R^2-NH_2 \qquad III$$

worin $R^2$ obige Bedeutung besitzt, umsetzt, oder

b) ein rektionsfähiges funktionelles Derivat einer Säure der allgemeinen Formel

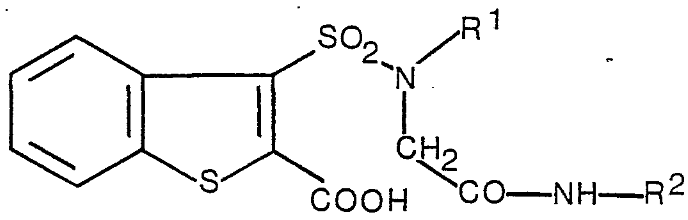

IV

worin $R^1$ und $R^2$ obige Bedeutung besitzen, cyclisiert, oder

c) eine Verbindung der allgemeinen Formel

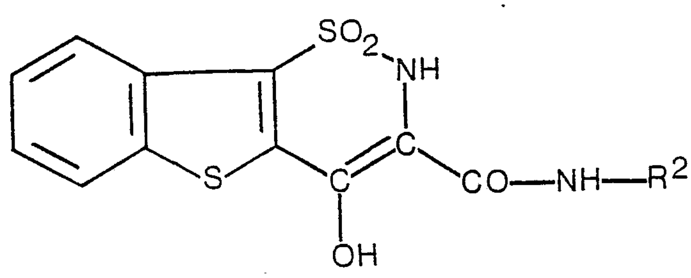

V

worin $R^2$ obige Bedeutung besitzt, entsprechend alkyliert, oder

d) eine Verbindung der allgemeinen Formel

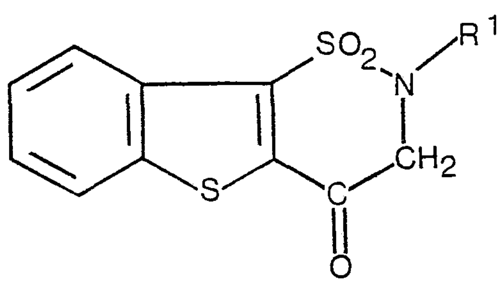

VI

worin $R^1$ obige Bedeutung besitzt, in Gegenwart einer starken Base mit einem Isocyanat der allgemeinen Formel

$$O = C = N - R^2 \qquad VII$$

worin $R^2$ obige Bedeutung besitzt, umsetzt, oder

e) ein Enamin der allgemeinen Formel

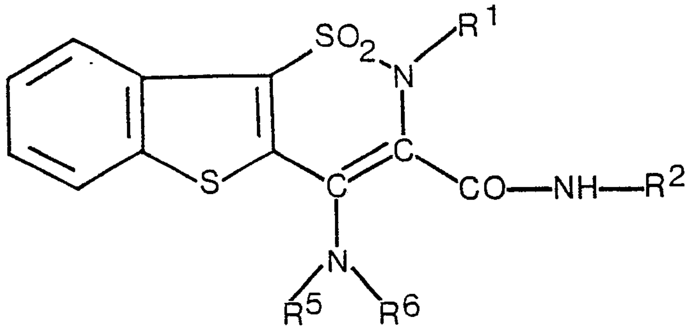

VIII

worin $R^1$ und $R^2$ obige Bedeutung besitzen, und $R^5$ und $R^6$ je niederes Alkyl oder zusammen mit dem Stickstoffatom Pyrrolin-1-yl, Pyrrolidin-1-yl, Piperidino, Morpholino oder 4-(nieder Alkyl)-piperazin-1-yl bedeuten, hydrolysiert, oder

f) eine Verbindung der allgemeinen Formel I in ein pharmazeutisch anwendbares Salz überführt.

Die Umsetzung nach Verfahrensaspekt a) kann in An- oder Abwesenheit eines inerten Lösungsmittels erfolgen. Als Lösungsmittel eignen sich Kohlenwasserstoffe, wie Benzol, Toluol, Xylol etc., halogenierte Kohlenwasserstoffe, wie Chloroform, Chlorbenzol, Methylenchlorid, Tetrachlorkohlenstoff etc., oder Dimethylformamid oder Dioxan. Die Umsetzung erfolgt vorzugsweise unter Erwärmen, wobei Schmelz- bzw. Rückflusstemperatur des Reaktionsgemisches besonders bevorzugt ist. In gewissen Fällen kann es angezeigt sein, das Amin der Formel III im Ueberschuss einzusetzen. Wenn R in Formel II niederes Alkoxy bedeutet, so geht man vorzugsweise so vor, dass der bei das Reaktion entstehende niedere Alkanol azeotrop aus dem Gemisch abdestilliert. Wenn R in Formel II einen Rest der Formel $-NR^3R^4$ bedeutet, so können $R^3$ und $R^4$ beide Wasserstoff oder eines Wasserstoff und das andere niederes Alkyl (z.B.

Methyl) oder beide niederes Alkyl (z.B. Methyl) sein; wenn $R^3$ und $R^4$ zusammen mit dem Stickstoffatom einen Heterocyclus bildet, so kommen in erster Linie 5- bis 7-gliedrige gesättigte heterocyclische Reste in Betracht, welche zusätzlich zum erwähnten Stickstoffatom noch ein Sauerstoffatom enthalten können, wie Pyrrolidin-1-yl, Piperidino, Morpholino o.dgl.

Nach dem Verfahrensaspekt b) gemäss vorliegender Erfindung wird ein reaktionsfähiges funktionelles Derivat einer Verbindung der Formel IV cyclisiert. Diese Cyclisation erfolgt in Gegenwart einer Base und vorzugsweise in Gegenwart eines Lösungsmittels bei einer Temperatur zwischen 0°C und Rückflusstemperatur des Reaktionsgemisches, vorzugsweise zwischen Raumtemperatur und 60°C. Als Basen kommen insbesondere Hydride, Amide oder Alkoholate von Alkalimetallen in Betracht. Als Lösungsmittel eignen sich aprotische und protische, wie Alkohole (z.B. Methanol und Aethanol), Aether (z.B. Dioxan) und Säureamide (z.B. Dimethylformamid). Zweckmässigerweise wird die Cyclisation so durchgeführt, dass man die Ausgangsverbindung der Formel IV im Lösungsmittel löst, mit der Base versetzt und das Reaktionsgemisch während 1 bis 4 Stunden entweder bei Raumtemperatur stehen lässt oder auf eine Temperatur bis zu 60°C erwärmt. Als reaktionsfähige funktionelle Derivate der Verbindungen der Formel IV eignen sich insbesondere deren niedere Alkylester, z.B. deren Methylester.

Nach dem Verfahrensaspekt c) wird eine Verbindung der Formel V alkyliert. Diese Alkylierung wird zweckmässigerweise so durchgeführt, dass man die Ausgangsverbindung der Formel V in einem aprotischen Lösungsmittel, wie Acetonitril, Dioxan oder Dimethylformamid, löst, mit einem Alkalimetallamid oder -hydrid in das Alkalimetallsalz überführt und dann durch Behandeln mit einem Alkylierungsmittel, insbesondere einem Alkylhalogenid oder -sulfat, in die entsprechende Verbindung der Formel I überführt. Temperatur und Druck sind für dieses Verfahren nicht kritisch, so dass die Reaktion der Einfachheit halber vorzugsweise bei Raumtemperatur und Atmosphärendruck durchgeführt wird.

Nach dem Verfahrensaspekt d) wird eine Verbindung der allgemeinen Formel VI in Gegenwart einer starken Base mit einem Isocyanat der allgemeinen Formel VII umgesetzt. Als starke Basen eignen sich Alkaliamide, Alkali- oder Erdalkalihydride sowie metallisches Alkali. Die Umsetzung erfolgt vorzugsweise unter einem Inertgas, z.B. Stickstoff, bei einer Temperatur zwischen 0°C-50°C, vorzugsweise bei Raumtemperatur, und in Gegenwart eines inerten Lösungsmittels, wie Toluol, Dioxan, Dimethylformamid, Dimethylsulfoxyd oder Hexamethylphosphorsäuretriamid (HMPT). Die Isocyanate der Formel VII sind entweder bekannt oder können in Analogie zur Herstellung der bekannten Vertreter synthetisiert werden.

Nach dem Verfahrensaspekt e) wird ein Enamin der allgemeinen Formel VIII hydrolysiert. Die Hydrolyse erfolgt vorzugsweise mit einer wässrigen Mineralsäure, wie Chlorwasserstoffsäure, Bromwasserstoffsäure, Jodwasserstoffsäure, Schwefelsäure, oder mit Trifluoressigsäure, wobei bei einer Temperatur zwischen 50°C und dem Siedepunkt des Reaktionsgemisches, vorzugsweise dem Siedepunkt des Reaktionsgemisches, gearbeitet wird. Bei dieser Hydrolyse dient die Säure gleichzeitig als Lösungsmittel.

Die Ausgangsverbindungen der Formel II für das Verfahren gemäss Variante a) können ausgehend von bekannten Produkten in an sich bekannter Weise hergestellt werden. Insbesondere können sie gemäss dem folgenden Reaktionsschema I, worin $R^1$, $R^3$ und $R^4$ die eingangs erwähnte Bedeutung besitzen und R' niederes Alkyl bedeutet, und gemäss den spezifischen Angaben in Beispielen 1 und 7 synthetisiert werden.

Reaktionsschema I

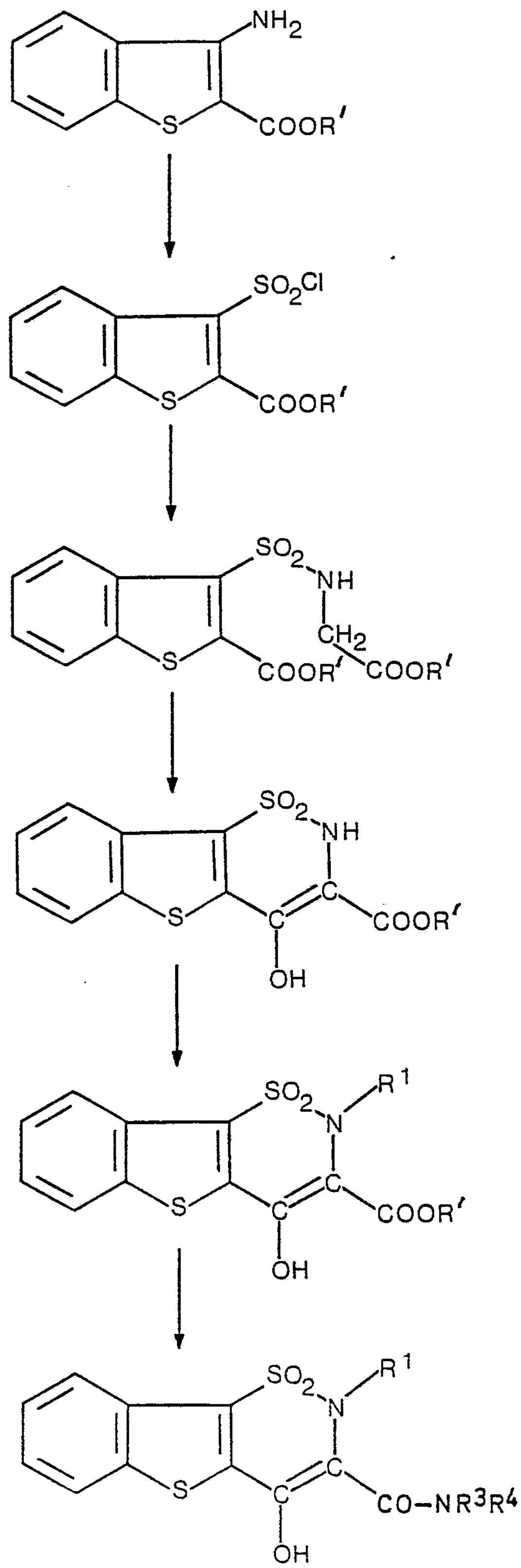

IX

X

XI

XII

II a

II b

Für Verfahrensaspekt b) als Ausgangsverbindungen verwendbare niedere Alkylester von Säuren der Formel IV können z.B. erhalten werden, indem man ein Amin der Formel III mit Chloracetylchlorid umsetzt und das erhaltene Produkt der Formel

$$Cl—CH_2—CO—NH—R^2 \qquad XIII$$

worin $R^2$ obige Bedeutung besitzt, mit einer Verbindung der Formel

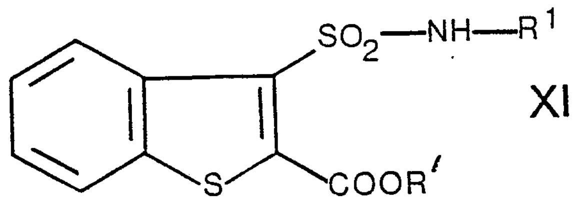

XIV

worin R', $R^1$ und $R^2$ obige Bedeutung besitzen, umsetzt oder indem man Verbindungen der Formel X mit Verbindungen der Formel

$$R^1\text{-NH-CH}_2\text{-CO-NH-}R^2 \qquad XV$$

worin $R^1$ und $R^2$ obige Bedeutung besitzen, umsetzt. Andere reaktionsfähige funktionelle Derivate von Säuren der Formel IV können in an sich bekannter Weise aus den erhaltenen Estern hergestellt werden. Verbindungen der Formel XIV können ihrerseits z.B. durch Umsetzung von Verbindungen der Formel X mit Aminen der Formel

$$R^1\text{-NH}_2 \qquad XVI$$

worin $R^1$ obige Bedeutung besitzt, hergestellt werden.

Für Verfahren c) verwendbare Ausgangsstoffe der Formel V können z.B. durch Umsetzen einer Verbindung der Formel XII mit einem Amin der Formel III erhalten werden,

wobei es zweckmässig ist das Stickstoffatom in 2-Stellung der Verbindung der Formel XII vor der Umsetzung mit dem Amin der Formel III zu schützen (beispielsweise mit einer geeigneten Aralkylgruppe, wie 4-Methoxybenzyl) und nach der erwähnten Umsetzung mit dem Amin der Formel III die Schutzgruppe wieder abzuspalten.

Für Verfahren d) verwendbare Ausgangsstoffe der Formel VI können z.B. dadurch hergestellt werden, dass man eine Verbindung der Formel IIa unter sauren Bedingungen hydrolysiert und decarboxyliert.

Die Herstellung der Ausgangsstoffe für Verfahren e) kann nach dem folgenden Reaktionsschema II, worin $R^1$, $R^2$, $R^5$ und $R^6$ die eingangs erwähnte Bedeutung besitzen, und nach den spezifischen Angaben in Beispiel 6 erfolgen.

Reaktionsschema II

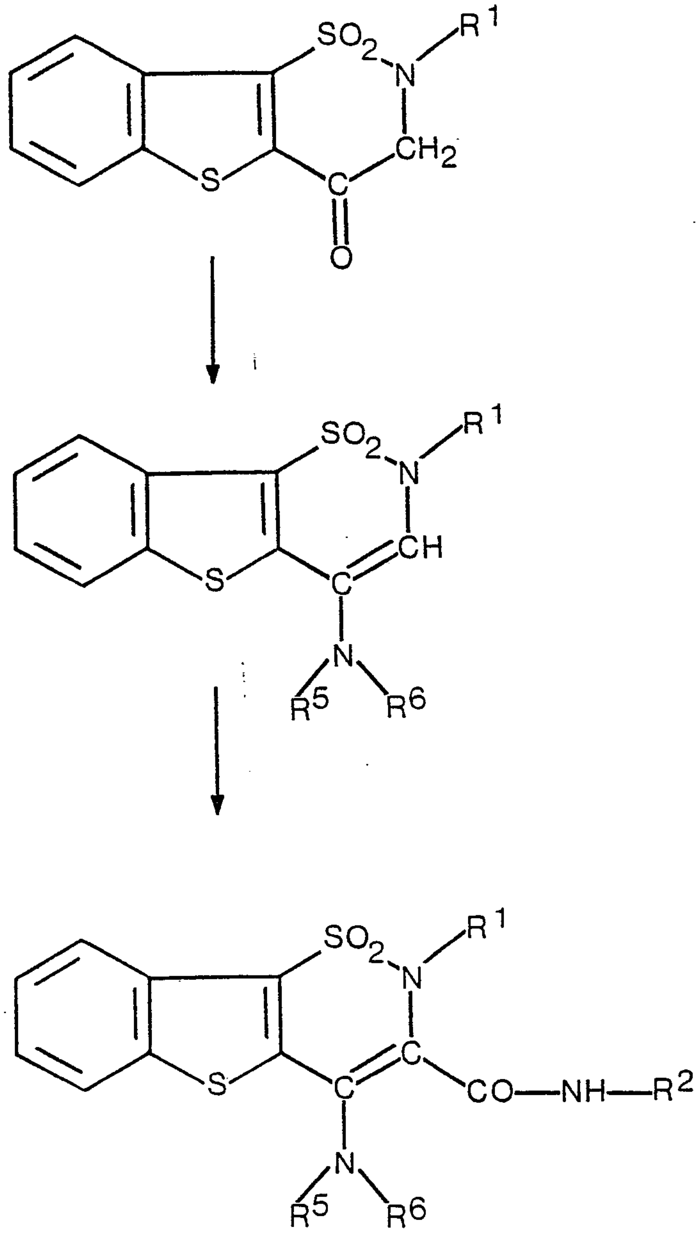

VI

XVII

VIII

Die Verbindungen der Formeln II, IV, V, VI und VIII sind ebenfalls Gegenstand der vorliegenden Erfindung.

Die Verbindungen der Formel I sind sauer und können mit entsprechenden Basen pharmazeutisch annehmbare Salze bilden. Als derartige Salze eignen sich z.B. Alkalimetallsalze, wie Lithium-, Natrium- oder Kaliumsalze; Erdalkalimetallsalze, wie Magnesium- oder Calciumsalze; sowie Salze mit Aminen, wie Triäthanolamin, Diäthylaminoäthanol, Triäthylamin, Trimethylamin oder Diäthylamin und dgl.. Verbindungen der Formel I mit einem basischen Heterocyclus $R^2$ können mit starken Säuren auch pharmazeutisch annehmbare Säureadditionssalze bilden, wozu insbesondere Mineralsäuren, wie Salzsäure in Frage kommen.

Die Verbindungen der allgemeinen Formel I sowie deren Salze haben eine antiinflammatorische, analgetische und antirheumatische Wirkung. Diese wertvollen pharmakologischen Eigenschaften können unter Verwendung von Standardmethoden bestimmt werden, beispielsweise im bekannten Kaolin-Pfotenoedemtest (an der Ratte). In diesem Test wird in der rechten Hinterpfote der Ratte durch intradermale Injektion von 0,1 ml einer 10%igen Kaolinsuspension (bolus alba) eine akute lokale Entzündung erzeugt. Die zu untersuchende Substanz wird auf oralem Wege verabreicht, und die folgenden Parameter werden gemessen:

1. Durchmesser der Pfote in mm (als Ausdruck der Heftigkeit der Entzündung);

2. Druck (in g) auf die Pfote (zur Ermittlung der Schmerzschwelle).

1/2 Stunde vor und 3 1/2 Stunden nach der Kaolininjektion wird die zu untersuchende Substanz verabreicht, und 4 Stunden nach der Kaolin-Injektion werden die oben erwähnten Parameter gemessen. Der oedemhemmende Effekt wird in Prozenten angegeben, basierend auf der Differenz der Oedemintensität zwischen unbehandelten und mit der zu untersuchenden Substanz behandelten Tieren, die antinoziceptive Aktivität durch die prozentuale Erhöhung der Schmerzschwelle.

In diesem Test zeigt das 4-Hydroxy-2-methyl-N-(2-pyridyl)-2H-[1]benzothieno[2,3-e]-1,2-thiazin-3-carboxamid-1,1-dioxid bei einer Dosierung von 1 mg/kg p.o. eine 24%ige Oedemhemmung und eine 66%ige Erhöhung der Schmerzschwelle.

Die Verbindungen der Formel I besitzen qualitativ eine ähnliche Wirkung wie Phenylbutazon, welches für seine therapeutische Verwendung und Eigenschaften bekannt ist. Ausserdem hemmen sie - wie dies in einem entsprechenden Standard-Test gezeigt werden kann - die Blutplättchenaggregation und haben demnach auch antithrombotische Eigenschaften.

Die Verbindungen der Formel I sowie deren Salze können als Heilmittel, z.B. in Form pharmazeutischer Präparate Verwendung finden, welche sie in Mischung mit einem für enterale oder parenterale Applikation geeigneten, pharmazeutischen, organischen oder anoranischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline usw. enthalten. Die pharmazeutischen Präparate können in fester Form, z.B. als Tabletten, Dragées, Suppositorien, Kapseln, in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer. Sie können auch noch andere therapeutisch wertvolle Stoffe enthalten.

Wie eingangs erwähnt, kann man Verbindungen der allgemeinen Formel I und pharmazeutisch anwendbare Salze davon erfindungsgemäss bei der Bekämpfung bzw. Verhütung von Krankheiten verwenden, insbesondere bei der Bekämpfung bzw. Verhütung von Entzündungen, Schmerzen, Rheuma und Thrombosen. Die Dosierung der Verbindungen der allgemeinen Formel I und ihrer pharmazeutisch anwendbaren Salze kann innerhalb ziemlich weiter Grenzen variieren und ist natürlich in jedem einzelnen Fall den individuellen Gegebenheiten anzupassen. Im allgemeinen dürfte beim Menschen eine orale Tagesdosis von etwa 5 bis etwa 100 mg, vorzugsweise etwa 10 bis etwa 30 mg, angemessen sein.

Wie eingangs erwähnt, sind Arzneimittel, enthaltend eine Verbindung der allgemeinen Formel I oder ein pharmazeutisch anwendbares Salz davon, ebenfalls Gegenstand der vorliegenden Erfindung, weiterhin auch ein Verfahren zur Herstellung solcher Arzneimittel, welches dadurch gekennzeichnet ist, dass man eine oder mehrere Verbindungen der allgemeinen Formel I oder pharmazeutisch akzeptable Salze davon und gegebenenfalls einen oder mehrere andere therapeutisch wertvolle Stoffe in eine galenische Dareichungsform bringt.

Die nachfolgenden Beispiele, in welchen alle Temperaturen in Celsiusgraden angegeben sind, erläutern die Erfindung.

## Beispiel 1

a) 5 g nach J. Org. Chem. 37, 3224 (1972) hergestelltes 3-Amino-2-carbomethoxy-benzothiophen werden bei 0° in 40 ml konz. Salzsäure suspendiert. Das Gemisch wird mit 20 ml Wasser verdünnt und 10 Minuten bei 0° gerührt. Bei -15° wird eine Lösung von 1,83 g Natriumnitrit in 15 ml Wasser zugetropft und dann 40 Minuten bei -15° gerührt. Bei -5° wird eine frisch hergestellte Suspension, erhalten durch Zusammengeben einer Lösung von 1,5 g Kupfer(II)chlorid in 3 ml Wasser und 50 ml einer 30%igen Lösung von Schwefeldioxid in Eisessig, schnell zugetropft. Dann wird ohne Kühlung 4 Stunden gerührt und die Suspension in 400 ml Wasser von 0° gegossen. Die entstandenen Kristalle werden abgesaugt, mit Wasser gewaschen und in Methylenchlorid gelöst. Die Lösung wird mit Natriumsulfat getrocknet und eingedampft. Der Rückstand wird aus Methylenchlorid unter Zusatz von Hexan umkristallisiert. Das erhaltene 2-Carbomethoxy-benzothiophen-3-sulfochlorid schmilzt bei 103-105°.

b) 10,3 g des gemäss a) erhaltenen Sulfochlorids und 4,45 g Glycinmethylester-hydrochlorid werden in 100 ml Pyridin über Nacht bei 23° gerührt. Das Lösungsmittel wird bei vermindertem Druck abdestilliert, worauf der Rückstand in 500 ml Methylenchlorid aufgenommen wird. Das Gemisch wird bei 0° mit 2N Salzsäure und Wasser gewaschen. Die organische Lösung wird mit Natriumsulfat getrocknet und eingedampft. Der Rückstand wird aus Methylenchlorid unter Zusatz von Hexan umkristallisiert. Der erhaltene N-(2-Carbomethoxy-benzothiophen-3-sulfonyl)glycinmethylester schmilzt bei 148-150°.

c) 1,55 g Natrium werden in 30 ml absolutem Methanol gelöst, worauf die Lösung unter Ausschluss von Feuchtigkeit bei 11 Torr eingedampft und der Rückstand bei 0,01 Torr getrocknet wird. Das erhaltene Natriummethylat wird mit

300 ml absolutem Toluol übergossen, worauf 10 g des gemäss b) erhaltenen Diesters zugegeben werden. Das Gemisch wird 6 Stunden bei 65° gerührt. Nach Abkühlen wird die dunkelrote Mischung in 800 ml Wasser von 0° eingerührt. Die wässerige Phase wird mit 2N Salzsäure sauer gestellt; die entstandenen Kristalle werden abgesaugt, mit Wasser gewaschen und bei 100° und 13 Torr getrocknet. Das erhaltene 3-Carbomethoxy-4-hydroxy-2H-[1]benzothieno[2,3-e]-1,2-thiazin-1,1-dioxid zeigt einen Schmelzpunkt von 240-242°.

d) 1,69 g einer 55%igen Natriumhydrid-Suspension in Mineralöl werden mit 130 ml absolutem Dimethylformamid übergossen, und mit 5,5 g des gemäss c) erhaltenen Benzothienothiazins versetzt. Das Reaktionsgemisch wird 1 Stunde bei 23° gerührt, dann tropfenweise mit 4,5 ml Methyljodid versetzt, über Nacht bei 23° gerührt und dann bei etwa 1 Torr eingedampft. Der Rückstand wird mit 400 ml Wasser von 0° gelöst. Durch Ansäuern mit 2N Salzsäure werden Kristalle erhalten, die abgesaugt und nach Waschen mit Wasser bei 0,01 Torr und 100° getrocknet werden. Das erhaltene 3-Carbomethoxy-4-hydroxy-2-methyl-2H-[1]benzothieno[2,3-e]-1,2-thiazin-1,1-dioxid zeigt einen Schmelzpunkt von 183-185°.

e) 5 g 3-Carbomethoxy-4-hydroxy-2-methyl-2H-[1]benzothieno[2,3-e]-1,2-thiazin-1,1-dioxid und 3 g 2-Aminopyridin werden mit 150 ml Xylol-Isomeren-Gemisch versetzt und so erwärmt, dass das entstehende Methanol azeotrop abdestilliert. Nach 6 Stunden lässt man auf Raumtemperatur abkühlen. Die erhaltenen Kristalle werden abgesaugt und mit Xylol-Gemisch gewaschen. Nach Umkristallisieren aus Dimethylformamid und Zusatz von Methanol erhält man 4-Hydroxy-2-methyl-N-(2-pyridyl)-2H-[1]benzothieno[2,3-e]-1,2-thiazin-3-carboxamid-1,1-dioxid in Form gelber Kristalle vom Schmelzpunkt 222-224°.

f) 774 mg 4-Hydroxy-2-methyl-N-(2-pyridyl)-2H-[1]benzo-

thieno[2,3-e]-1,2-thiazin-3-carboxamid-1,1-dioxid werden in 10 ml Methanol suspendiert, worauf 20 ml 0,1N Natronlauge zugegeben werden. Nach rund 20 Minuten Rühren bei Raumtemperatur entsteht eine klare Lösung, die bei 11 Torr eingedampft wird. Der Rückstand wird in wenig Methanol gelöst. Durch Zugabe von absolutem Diäthyläther wird das Mono-Natrium-Salz des 4-Hydroxy-2-methyl-N-(2-pyridyl)-2H-[1]benzothieno[2,3-e]-1,2-thiazin-3-carboxamid-1,1-dioxids kristallin mit 0,69% Kristallwasser und einem Schmelzpunkt von 206-208° erhalten.

## Beispiel 2

In analoger Weise wie in Absatz e) von Beispiel 1 beschrieben wird aus 1,63 g 3-Carbomethoxy-4-hydroxy-2-methyl-2H-[1]benzothieno[2,3-e]-1,2-thiazin-1,1-dioxid und 1,22 g 6-Amino-2-methyl-pyridin in 50 ml o-Xylol das 4-Hydroxy-2-methyl-N-(6-methyl-pyridyl-2)-2H-[1]benzothieno-[2,3-e]-1,2-thiazin-3-carboxamid-1,1-dioxid vom Schmelzpunkt 250-251° erhalten.

## Beispiel 3

a) 12,2 g 2-Carbomethoxy-benzothiophen-3-sulfochlorid werden bei Raumtemperatur unter Rühren in 200 ml Pyridin eingetragen, worauf 10 g Sarkosinanilid-hydrochlorid zugegeben werden. Man rührt über Nacht (16 Stunden) weiter und dampft bei 11 Torr ein. Der Rückstand wird mit Methylenchlorid übergossen und das Gemisch mit 2N Salzsäure bei 0° ausgeschüttelt. Die organische Phase wird mit Wasser gewaschen, mit Natriumsulfat getrocknet und bei 11 Torr eingedampft. Der Rückstand kristallisiert aus Methylenchlorid durch Zusatz von Petroläther. Der 3-[N-(Phenyl-carbamoylmethyl)-N-methyl]-sulfamoyl-benzothieno-2-carbonsäuremethylester schmilzt bei 114-117°.

b) 1,05 g der erhaltenen Verbindung werden unter Feuchtigkeitsausschluss in 50 ml absolutem Pyridin gelöst, worauf 0,45 g reines Natriummethylat zugegeben werden. Man rührt 2 Stunden bei 50° und dampft dann bei 11 Torr ein. Der Rückstand wird in 100 ml Wasser aufgenommen und dreimal mit je 40 ml Methylenchlorid ausgeschüttelt, worauf die Extrakte mit 50 ml Wasser gewaschen werden. Die wässrigen Lösungen werden vereinigt, mit etwa Methanol verdünnt, mit Kohle entfärbt, mit 2N Salzsäure auf pH 2 angesäuert und das Methanol bei 11 Torr entfernt. Der Niederschlag wird mit Wasser verrührt und dann in Methylenchlorid gelöst, worauf man die Lösung mit 2 g Kieselgel verrührt und filtriert. Das Filtrat wird eingedampft und der Rückstand mit Diäthyläther verrührt. Man erhält nach Filtration und Trocknen bei 11 Torr 4-Hydroxy-2-methyl-N-phenyl-2H-[1]-benzothieno[2,3-e]-1,2-thiazin-3-carboxamid-1,1-dioxid vom Schmelzpunkt 261-263°.

Beispiel 4

a) 935 mg 3-Carbomethoxy-4-hydroxy-2H-[1]benzothieno-[2,3-e]-1,2-thiazin-1,1-dioxid werden in 10 ml absolutem Dimethylformamid gelöst, worauf 290 mg einer 55% Natriumhydrid-Dispersion in Paraffin zugegeben werden. Die Suspension wird 1 Stunde bei Raumtemperatur gerührt. Dann wird eine Lösung von 730 mg 4-Methoxybenzylbromid in 5 ml absolutem Dimethylformamid innerhalb von 30 Minuten zugetropft, worauf das Gemisch 3 Stunden bei Raumtemperatur weitergerührt und dann unter Rühren in 100 ml eiskaltes Wasser gegossen wird. Hierauf wird mit konz. Salzsäure auf pH 2 angesäuert. Der entstandene Niederschlag wird abgesaugt und in Methylenchlorid gelöst; die Lösung wird mit Natriumsulfat getrocknet, filtriert und eingedampft. Der Rückstand kristallisiert aus Methylenchlorid nach Zusatz von Petroläther. Das erhaltene 3-Carbomethoxy-4-hydroxy-2-(4-methoxybenzyl)-2H-[1]benzothieno[2,3-e]-1,2-thiazin-1,1-dioxid schmilzt bei 163-164°.

b) 1 g der erhaltenen Verbindung und 550 mg 2-Aminopyridin werden in 30 ml o-Xylol gelöst. Das Gemisch wird unter Rühren 6 Stunden auf 150° (Badtemperatur) erhitzt, wobei das entstehende Methanol azeotrop abdestilliert. Das Reaktionsgemisch wird dann bei 11 Torr eingedampft. Der Rückstand wird mit einer Mischung aus 15 ml Methylenchlorid und 15 ml Aether aufgekocht. Man lässt erkalten, worauf der entstandene Niederschlag abgesaugt und aus Methylenchlorid unter Zugabe von Petroläther umkristallisiert wird. Das erhaltene 4-Hydroxy-2-(4-methoxybenzyl)-N-(2-pyridyl)-2H-[1]benzothieno[2,3-e]-1,2-thiazin-3-carboxamid-1,1-dioxid schmilzt bei 188-190°.

c) 400 mg der erhaltenen Verbindung werden in 5 ml Trifluoressigsäure gelöst, worauf 0,9 ml Anisol zugegeben werden. Das Gemisch wird 16 Stunden bei 50° gerührt und dann bei 11 Torr eingedampft. Der Rückstand wird mit Aether verrührt und filtriert. Der Filterkuchen wird in verdünnter Natronlauge gelöst, worauf man mit Kohle entfärbt, filtriert und das Filtrat mit 2N Salzsäure neutralisiert. Die Kristalle werden abgesaugt, mit Wasser gewaschen und 4 Stunden bei 100° und 13 Torr getrocknet. Das erhaltene 4-Hydroxy-N-(2-pyridyl)-2H-[1]benzothieno[2,3-e]-1,2-thiazin-3-carboxamid-1,1-dioxid schmilzt bei 240-242°.

d) 790 mg der erhaltenen Verbindung werden in 10 ml absolutem Dimethylformamid gelöst, worauf 93 mg einer 55% Natriumhydrid-Dispersion in Paraffin zugesetzt werden. Das Gemisch wird 45 Minuten bei Raumtemperatur gerührt, worauf man bei 0° 2 ml Methyljodid zutropft und 4 Stunden bei Raumtemperatur nachrührt. Das Gemisch wird in Eiswasser eingerührt und mit 2N Salzsäure neutralisiert. Der Niederschlag wird abgesaugt und in Chloroform unter Zusatz von Methanol gelöst. Die Lösung wird über Natriumsulfat getrocknet und über 20 g Kieselgel filtriert. Das Eluat wird bei 11 Torr eingedampft und der Rückstand mit Acetonitril verrührt. Die erhaltenen Kristalle des

4-Hydroxy-2-methyl-N-(2-pyridyl)-2H-[1]benzothieno[2,3-e]-1,2-thiazin-3-carboxamid-1,1-dioxids schmelzen, nach Absaugen und Trocknen, bei 222-224°.

## Beispiel 5

a) 10 g 3-Carbomethoxy-4-hydroxy-2-methyl-2H-[1]benzothieno[2,3-e]-1,2-thiazin-1,1-dioxid werden einem Gemisch von 200 ml Eisessig und 200 ml konz. Salzsäure zugesetzt, worauf 18 Stunden bei einer Oelbadtemperatur von 140° unter Rückfluss gekocht wird. Der Kolbeninhalt wird bei 11 Torr eingedampft und der Rückstand in 300 ml Wasser suspendiert. Die erhaltenen Kristalle werden abgesaugt und in 300 ml Methylenchlorid gelöst. Die Lösung wird mit Natriumsulfat getrocknet, über 50 g Kieselgel filtriert und mit Petroläther versetzt, wonach 3,4-Dihydro-2-methyl-4-oxo-2H-[1]benzothieno[2,3-e]-1,2-thiazin-1,1-dioxid auskristallisiert. Nach 12 Stunden Trocknen über Phosphorpentoxid bei 50° und 0,01 Torr zeigt das Produkt einen Schmelzpunkt von 184-186°.

b) 2,67 g 3,4-Dihydro-2-methyl-4-oxo-2H-[1]benzothieno-[2,3-e]-1,2-thiazin-1,1-dioxid werden in 40 ml absolutem Dimethylformamid gelöst, worauf 1,2 ml Phenylisocyanat zugegeben werden. Die erhaltene Lösung wird bei 4° innerhalb von 30 Minuten zu einer Suspension von 600 mg einer 55% Natriumhydrid-Dispersion in Paraffin in 10 ml Dimethylformamid getropft. Das Gemisch wird 1 Stunde bei 4° und anschliessend 2 Stunden bei 27° weitergerührt und dann in 400 ml Eiswasser eingerührt. Die erhaltene Lösung wird mit konz. Salzsäure sauer gestellt. Der Niederschlag wird abgesaugt, mit Wasser gewaschen und in Methylenchlorid gelöst, worauf die Lösung mit Natriumsulfat getrocknet und eingedampft wird. Der Rückstand wird mit 30 ml Methanol verrührt. Die ungelösten Teile werden mit Acetonitril digeriert, abgesaugt und 4 Stunden bei 100° und 11 Torr getrocknet. Das erhaltene 4-Hydroxy-2-methyl-N-phenyl-2H-[1]benzothieno[2,3-e]-thiazin-3-carboxamid-1,1-dioxid schmilzt bei 261-263°.

Beispiel 6

a) 1,4 g 3,4-Dihydro-2-methyl-4-oxo-2H-[1]benzothieno-[2,3-e]-1,2-thiazin-1,1-dioxid, 0,9 ml Pyrrolidin und 10 mg p-Toluolsulfosäure-monohydrat werden in 20 ml Benzol 6 Stunden am Wasserabscheider gekocht. Die Lösung wird bei 11 Torr eingedampft, worauf man den Rückstand in Methylenchlorid löst und über Kieselgel filtriert. Durch Zusatz von Petroläther kristallisiert das 2-Methyl-4-(1-pyrrolidinyl)-2H-[1]benzothieno[2,3-e]-1,2-thiazin-1,1-dioxid aus. Schmelzpunkt 164-166°.

b) 2,1 g der erhaltenen Verbindung und 1,3 ml Triäthylamin werden in 50 ml absolutem Tetrahydrofuran gelöst. Bei 0-5° werden 4 ml einer 20% Lösung von Phosgen in Toluol zugetropft, worauf man 3 Stunden auf 50° erwärmt. Nach Zugabe von 1,3 ml Triäthylamin und 880 mg 2-Aminopyridin wird nochmals 2 Stunden bei 50° gerührt. Das Gemisch wird nach dem Erkalten in 180 ml Wasser eingerührt und das Ganze mit Methylenchlorid ausgeschüttelt. Ein unlöslicher Niederschlag wird abfiltriert (Smp. 325°). Die Methylenchloridlösung wird mit Natriumsulfat getrocknet und über 100 g Kieselgel chromatographiert. Das 2-Methyl-N-(2-pyridyl)-4-(1-pyrrolidinyl)-2H-[1]benzothieno[2,3-e]-1,2-thiazin-3-carboxamid-1,1-dioxid schmilzt bei 183-185°.

c) Die erhaltene Verbindung wird in 2 ml 2N Salzsäure gelöst und 10 Minuten auf 100° erwärmt. Die Lösung wird mit 2N Natriumcarbonatlösung neutralisiert und mit Methylenchlorid ausgeschüttelt. Die organische Phase wird über Natriumsulfat getrocknet und eingedampft. Das 4-Hydroxy-2-methyl-N-(2-pyridyl)-2H-[1]benzothieno[2,3-e]-1,2-thiazin-3-carboxamid-1,1-dioxid wird durch Verrühren mit Aether erhalten und schmilzt bei 222°.

Beispiel 7

a) 2 g 3-Carbomethoxy-4-hydroxy-2-methyl-2H-[1]benzothieno[2,3-e]-1,2-thiazin-1,1-dioxid werden in 100 ml o-Xylol gelöst und bei 150° gerührt. Während 8 Stunden wird Ammoniak eingeleitet, worauf abgekühlt und abgesaugt wird. Der Rückstand wird mit Methylenchlorid verrührt, abgesaugt und bei 11 Torr getrocknet. Das 4-Hydroxy-2-methyl-2H-[1]benzothieno[2,3-e]-1,2-thiazin-3-carboxamid-1,1-dioxid schmilzt bei 247-248° unter Zersetzung.

b) 1,5 g der erhaltenen Verbindung und 850 mg 2-Aminopyridin werden in 100 ml o-Xylol während 20 Stunden unter Rückfluss gekocht (Badtemperatur 150°). Das Gemisch wird eingedampft; der Rückstand wird in Chloroform gelöst, worauf man über Kieselgel filtriert. Das 4-Hydroxy-2-methyl-N-(2-pyridyl)-2H-[1]benzothieno[2,3-e]-1,2-thiazin-3-carboxamid-1,1-dioxid schmilzt nach Verreiben mit Acetonitril und Trocknen bei 224-225,5°.

Beispiel A

Es werden in üblicher Weise Suppositorien folgender Zusammensetzung hergestellt:

| | Pro Suppositorium |
|---|---|
| 4-Hydroxy-2-methyl-N-(2-pyridyl)-2H-[1]benzothieno[2,3-e]-1,2-thiazin-3-carboxamid-1,1-dioxid | 0,020 g |
| Hydriertes Kokosnussöl | 1,235 g |
| Carnaubawachs | 0,045 g |

Beispiel B

Es werden in üblicher Weise Tabletten folgender Zusammensetzung hergestellt:

| | Pro Tablette |
|---|---|
| 4-Hydroxy-2-methyl-N-(2-pyridyl)-2H-[1]benzothieno[2,3-e]-1,2-thiazin-3-carboxamid-1,1-dioxid | 10,0 mg |
| Lactose | 80,0 mg |
| Maisstärke | 9,0 mg |
| Magnesiumstearat | 1,5 mg |

Beispiel C

Es werden in üblicher Weise Kapseln folgender Zusammensetzung hergestellt:

| | Pro Kapsel |
|---|---|
| 4-Hydroxy-2-methyl-N-(2-pyridyl)-2H-[1]benzothieno[2,3-e]-1,2-thiazin-3-carboxamid-1,1-dioxid | 10,0 mg |
| Lactose | 165,0 mg |
| Maisstärke | 30,0 mg |
| Talk | 5,0 mg |

## Patentansprüche

1. Benzothienothiazinderivate der allgemeinen Formel

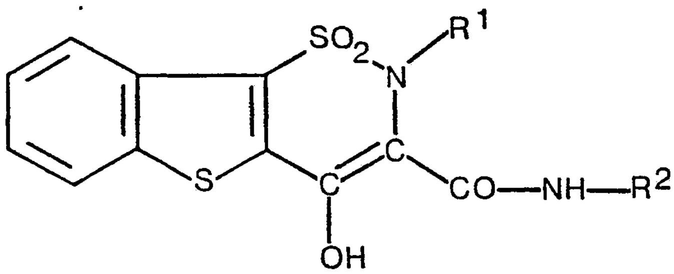

I

worin $R^1$ niederes Alkyl und $R^2$ den Rest eines gegebenenfalls durch eine oder zwei niedere Alkylgruppen substituierten aromatischen Heterocyclus mit 1 bis 4 Heteroatomen oder einen gegebenenfalls durch Halogen, Hydroxy, niederes Alkyl, Trifluormethyl, Nitro oder niederes Alkoxy substituierten Phenyl- oder Benzylrest bedeuten,

sowie pharmazeutisch anwendbare Salze davon.

2. Verbindungen gemäss Anspruch 1, worin $R^2$ 2-Thiazolyl, 4-Methyl-2-thiazolyl, 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl, Phenyl, 4-Hydroxyphenyl, 3-Tolyl, 3-Chlorphenyl, 4-Fluorphenyl, 3-Trifluormethylphenyl, 2,4-Dichlorphenyl, 4-Bromphenyl, 4-Chlorphenyl, 4-Nitrophenyl, 2-Tolyl, 2,5-Dichlorphenyl, 4-Nitro-2-tolyl, 4-Jodphenyl, 4-n-Butylphenyl, Benzyl oder 2-Chlorphenyl bedeutet.

3. Verbindungen gemäss Anspruch 1 oder 2, worin $R^1$ Methyl bedeutet.

4. Verbindungen gemäss Anspruch 1 oder 3, worin $R^2$ einen gegebenenfalls durch niederes Alkyl monosubstituierten

Pyridylrest bedeutet.

5. Verbindungen gemäss Anspruch 4, worin $R^2$ 2-Pyridyl bedeutet.

6. 4-Hydroxy-2-methyl-N-(2-pyridyl)-2H-[1]benzothieno-[2,3-e]-1,2-thiazin-3-carboxamid-1,1-dioxid.

7. 4-Hydroxy-2-methyl-N-(6-methyl-pyridyl-2)-2H-[1]-benzothieno[2,3-e]-1,2-thiazin-3-carboxamid-1,1-dioxid und 4-Hyroxy-2-methyl-N-phenyl-2H-[1]benzothieno[2,3-e]-1,2-thiazin-3-carboxamid-1,1-dioxid.

8. Verbindungen der allgemeinen Formel

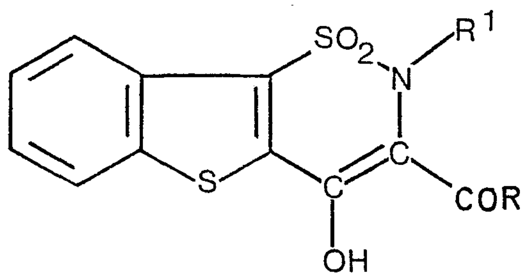

II

worin $R^1$ die in Anspruch 1 angegebene Bedeutung besitzt und R niederes Alkoxy oder einen Rest der Formel $-NR^3R^4$ bedeutet, wobei $R^3$ und $R^4$ je Wasserstoff oder niederes Alkyl oder zusammen mit dem Stickstoffatom einen Heterocyclus bedeuten.

9. Reaktionsfähige funktionelle Derivate von Säuren der allgemeinen Formel

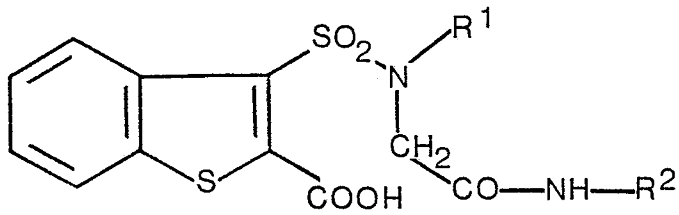

IV

worin $R^1$ und $R^2$ die in Anspruch 1 angegebene Bedeutung besitzen.

10. Verbindungen der allgemeinen Formel

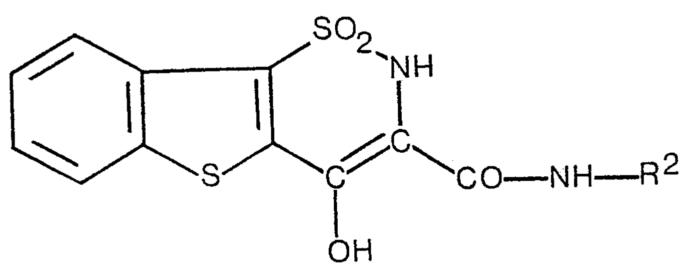

V

worin $R^2$ die in Anspruch 1 angegebene Bedeutung besitzt.

11. Verbindungen der allgemeinen Formel

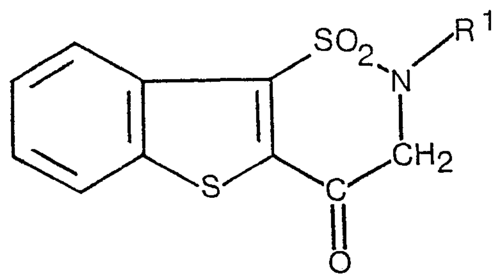

VI

worin $R^1$ die in Anspruch 1 angegebene Bedeutung besitzen.

12. Verbindungen der allgemeinen Formel

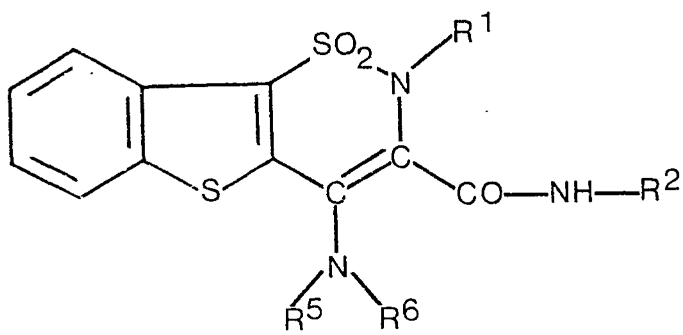

VIII

worin $R^1$ und $R^2$ die in Anspruch 1 angegebene Bedeutung besitzen und $R^5$ und $R^6$ je niederes Alkyl oder zusammen mit dem Stickstoffatom Pyrrolin-1-yl, Pyrrolidin-1-yl, Piperidino, Morpholino oder 4-(nieder Alkyl)-piperazin-1-yl bedeuten.

13. Thiazinderivate gemäss einem der Ansprüche 1 bis 7 als pharmazeutische Wirkstoffe.

14. Thiazinderivate gemäss einem der Ansprüche 1 bis 7 als antiinflammatorische, analgetische, antirheumatische und antithrombotische Wirkstoffe.

15. Verfahren zur Herstellung von Thiazinderivaten gemäss einem der Ansprüche 1 bis 7, dadurch gekennzeichnet, dass man

a) eine Verbindung der allgemeinen Formel

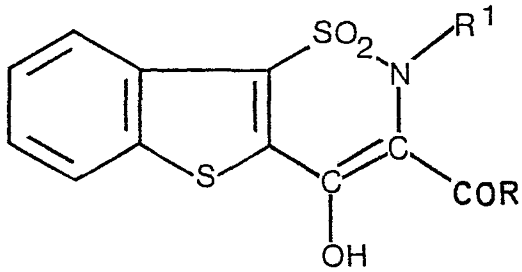

II

worin $R^1$ die in Anspruch 1 angegebene Bedeutung besitzt und R niederes Alkoxy oder einen Rest der Formel $-NR^3R^4$ bedeutet, wobei $R^3$ und $R^4$ je Wasserstoff oder niederes Alkyl oder zusammen mit dem Stickstoffatom einen Heterocyclus bedeuten,

mit einem Amin der allgemeinen Formel

$R^2-NH_2$ III

worin $R^2$ die in Anspruch 1 angegebene Bedeutung besitzt,

umsetzt, oder

b) ein reaktionsfähiges funktionelles Derivat einer Säure der allgemeinen Formel

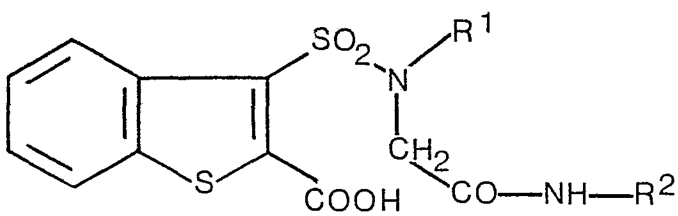

IV

worin $R^1$ und $R^2$ die in Anspruch 1 angegebene Bedeutung besitzen,

cyclisiert, oder

c) eine Verbindung der allgemeinen Formel

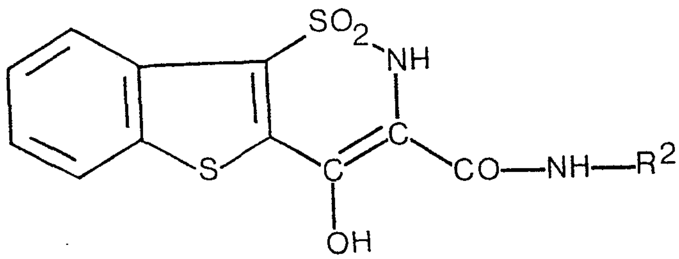

V

worin $R^2$ die in Anspruch 1 angegebene Bedeutung besitzt,

entsprechend alkyliert, oder

d) eine Verbindung der allgemeinen Formel

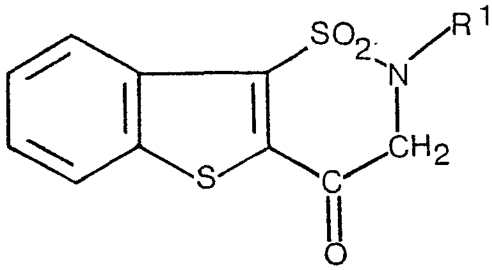

VI

worin $R^1$ die in Anspruch 1 angegebene Bedeutung besitzt,

in Gegenwart einer starken Base mit einem Isocyanat der allgemeinen Formel

$$O = C = N - R^2 \qquad VII$$

worin $R^2$ die in Anspruch 1 angegebene Bedeutung besitzt,

umsetzt, oder

e) ein Enamin der allgemeinen Formel

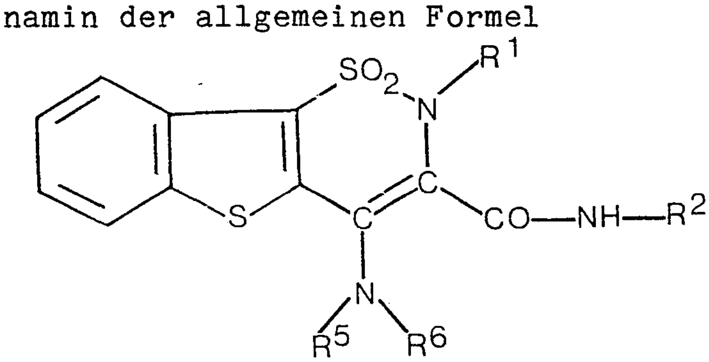

VIII

worin $R^1$ und $R^2$ die in Anspruch 1 angegebene Bedeutung besitzen, und $R^5$ und $R^6$ je niederes Alkyl oder zusammen mit dem Stickstoffatom Pyrrolin-1-yl, Pyrrolidin-1-yl, Piperidino, Morpholino oder 4-(nieder Alkyl)-piperazin-1-yl bedeuten,

hydrolysiert, oder

f) eine Verbindung der allgemeinen Formel I in ein pharmazeutisch anwendbares Salz überführt.

16. Arzneimittel, enthaltend ein Thienodiazinderivat gemäss einem der Ansprüche 1 bis 7.

17. Antiinflammatorisches, analgetisches, antirheumatisches und antithrombotisches Mittel, enthaltend ein Thienothiazinderivat gemäss einem der Ansprüche 1 bis 7.

18. Verwendung eines Thiazinderivates gemäss einem der Ansprüche 1 bis 7 bei der Bekämpfung bzw. Verhütung von Krankheiten.

19. Verwendung eines Thiazinderivates gemäss einem der Ansprüche 1 bis 7 bei der Bekämpfung bzw. Verhütung von Entzündungen, Schmerzen, Rheuma und Thrombosen.

****

## Patentansprüche für Oesterreich

1. Verfahren zur Herstellung von Benzothienothiazinderivaten der allgemeinen Formel

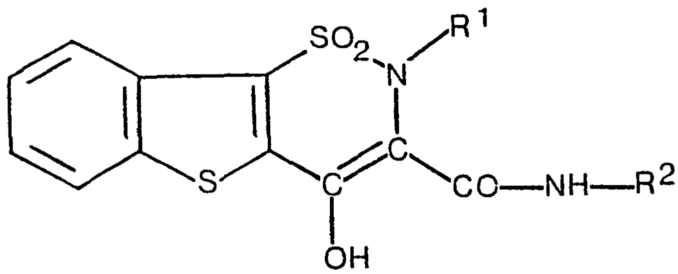

I

worin $R^1$ niederes Alkyl und $R^2$ den Rest eines gegebenenfalls durch eine oder zwei niedere Alkylgruppen substituierten aromatischen Heterocyclus mit 1 bis 4 Heteroatomen oder einen gegebenenfalls durch Halogen, Hydroxy, niederes Alkyl, Trifluormethyl, Nitro oder niederes Alkoxy substituierten Phenyl- oder Benzylrest bedeuten,

sowie von pharmazeutisch anwendbaren Salzen davon, dadurch gekennzeichnet, dass man

a) eine Verbindung der allgemeinen Formel

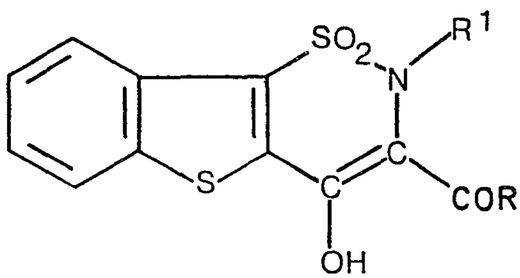

II

worin $R^1$ obige Bedeutung besitzt und R niederes Alkoxy oder einen Rest der Formel $-NR^3R^4$ bedeutet, wobei $R^3$ und $R^4$ je Wasserstoff oder niederes Alkyl oder zusammen mit dem Stickstoffatom einen Heterocyclus bedeuten,

mit einem Amin der allgemeinen Formel

$R^2-NH_2$ III

worin $R^2$ obige Bedeutung besitzt, umsetzt, oder

b) ein reaktionsfähiges funktionelles Derivat einer Säure der allgemeinen Formel

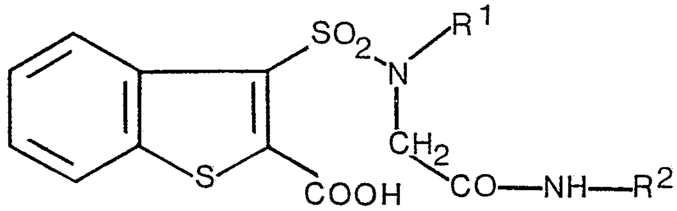

IV

worin $R^1$ und $R^2$ obige Bedeutung besitzen, cyclisiert, oder

c) eine Verbindung der allgemeinen Formel

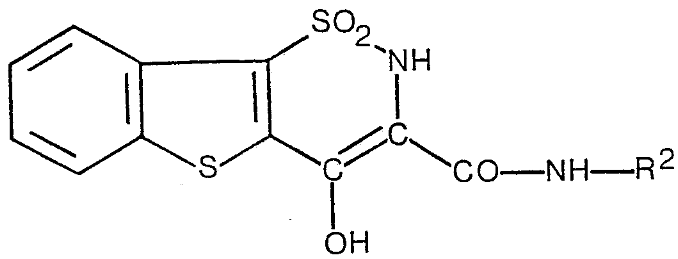

V

worin $R^2$ obige Bedeutung besitzt, entsprechend alkyliert, oder

d) eine Verbindung der allgemeinen Formel

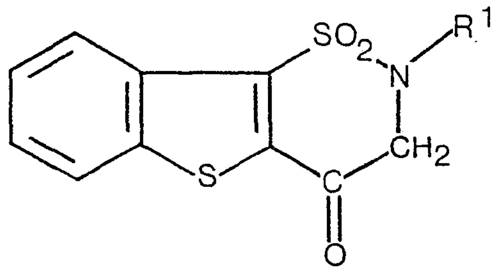

VI

worin $R^1$ obige Bedeutung besitzt, in Gegenwart einer starken Base mit einem Isocyanat der allgemeinen Formel

$$O = C = N - R^2 \qquad VII$$

worin $R^2$ obige Bedeutung besitzt, umsetzt, oder

e) ein Enamin der allgemeinen Formel

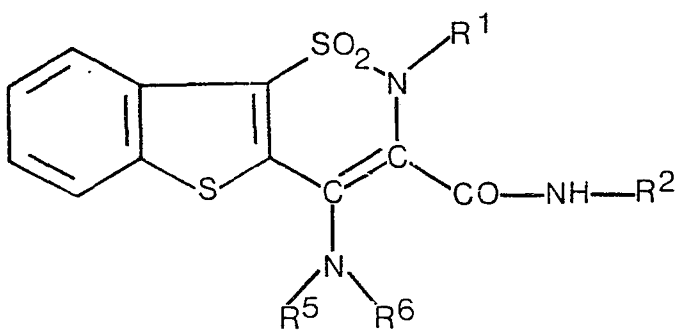

VIII

worin $R^1$ und $R^2$ obige Bedeutung besitzen, und $R^5$ und $R^6$ je niederes Alkyl oder zusammen mit dem Stickstoffatom Pyrrolin-1-yl, Pyrrolidin-1-yl, Piperidino, Morpholino oder 4-(nieder Alkyl)-piperazin-1-yl bedeuten, hydrolysiert, oder

f) eine Verbindung der allgemeinen Formel I in ein pharmazeutisch anwendbares Salz überführt.

2. Verfahren gemäss Anspruch 1, dadurch gekennzeichnet, dass man nach Varianten b) bis f) arbeitet oder dass man nach Variante a) arbeitet, wobei R in Formel II niederes Alkoxy bedeutet.

3. Verfahren gemäss Anspruch 1 oder 2, dadurch gekennzeichnet, dass $R^2$ 2-Thiazolyl, 4-Methyl-2-thiazolyl, 4,5-Dimethyl-2-thiazolyl, 5-Methyl-1,3,4-thiadiazolyl, 2-Pyrazinyl, 2-Pyrimidinyl, 1,2,4-Triazin-3-yl, 2-Pyridyl, 3-Pyridyl, 4-Pyridyl, 3-Methyl-2-pyridyl, 4-Methyl-2-pyridyl, 5-Methyl-2-pyridyl, 6-Methyl-2-pyridyl, 4,6-Dimethyl-2-pyridyl, 5-Isoxazolyl, 5-Methyl-3-isoxazolyl, 3,4-Dimethyl-5-isoxazolyl, 2,6-Dimethyl-4-pyrimidinyl, 1,2,3,4-Tetrazol-5-yl, Phenyl, 4-Hydroxyphenyl, 3-Tolyl, 3-Chlorphenyl, 4-Fluorphenyl, 3-Trifluormethylphenyl, 2,4-Dichlorphenyl, 4-Bromphenyl, 4-Chlorphenyl, 4-Nitrophenyl, 2-Tolyl, 2,5-Dichlorphenyl, 4-Nitro-2-tolyl, 4-Jodphenyl, 4-n-Butylphenyl, Benzyl oder 2-Chlorphenyl bedeutet.

4. Verfahren gemäss einem der Ansprüche 1 bis 3, dadurch gekennzeichnet, dass $R^1$ Methyl bedeutet.

5. Verfahren gemäss einem der Ansprüche 1, 2 und 4, dadurch gekennzeichnet, dass $R^2$ einen gegebenenfalls durch niederes Alkyl monosubstituierten Pyridylrest bedeutet.

6. Verfahren gemäss Anspruch 5, dadurch gekennzeichnet, dass $R^2$ 2-Pyridyl bedeutet.

7. Verfahren gemäss Anspruch 6, dadurch gekennzeichnet, dass man 4-Hydroxy-2-methyl-N-(2-pyridyl)-2H-[1]benzothieno[2,3-e]-1,2-thiazin-3-carboxamid-1,1-dioxid herstellt.

8. Verfahren gemäss Anspruch 5, dadurch gekennzeichnet, dass man 4-Hydroxy-2-methyl-N-(6-methyl-pyridyl-2)-2H-[1]benzothieno[2,3-e]1,2-thiazin-3-carboxamid-1,1-dioxid herstellt.

9. Verfahren gemäss Anspruch 4, dadurch gekennzeichnet, dass man 4-Hydroxy-2-methyl-N-phenyl-2H-[1]benzothieno-[2,3-e]-1,2-thiazin-3-carboxamid-1,1-dioxid herstellt.